# EUROPEAN PATENT APPLICATION

(11) **EP 4 431 105 A1**
(43) Date of publication of application: **18.09.2024**
(21) Application number: 22892849.5
(22) Date of filing: 10.11.2022
(51) Int. Cl.: A61K 36/05, A23K 10/30, A23L 33/10, A61K 8/9722, A61P 37/04, A61P 43/00, A61Q 19/00, C12N 1/12

(54) **T-CELL DIFFERENTIATION REGULATING AGENT AND COMPOSITION**

(30) Priority: 11.11.2021 JP 2021183874
(71) Applicant: KJ Bio Co., Ltd., Amakusa-shi, Kumamoto 863-2424 (JP); TOKAI UNIVERSITY EDUCATIONAL SYSTEM, Tokyo 151-0063 (JP)
(72) Inventor: KUNO, Hitoshi, Kariya-shi, Aichi 448-8661 (JP); KANNO, Akiko, Kariya-shi, Aichi 448-8661 (JP); KOMATSU, Satoko, Kariya-shi, Aichi 448-8661 (JP); KAMETANI, Yoshie, Isehara-shi, Kanagawa 259-1193 (JP); OHSHIMA, Shino, Isehara-shi, Kanagawa 259-1193 (JP); OHNO, Yusuke, Isehara-shi, Kanagawa 259-1193 (JP)
(74) Representative: Godemeyer Blum Lenze Patentanwälte Partnerschaft mbB - werkpatent
(86) International application number: PCT/JP2022/041870
(87) International publication number: WO 2023/085351

(57) **Abstract**

It is an object of the present invention to provide a means capable of appropriately regulating differentiation of T cells, in order to avoid overactivation of T cells after stimulation of the T cells with superantigens or the like and the subsequent decrease in the immune functions (exhaustion of the immune system). The present invention relates to a T cell differentiation-regulating agent, comprising, as an active ingredient, an algal body of microalgae belonging to the genus Coccomyxa, a dry powder thereof, or an extract thereof.

## Description

### Technical Field

The present invention relates to a T cell differentiation-regulating agent and a composition.

### Background Art

It has been known that *Staphylococcus aureus* enterotoxin comprises superantigens and causes toxic shock syndrome (TSS) to patients affected with enterotoxin (Non-Patent Document 1). These superantigens crosslink the T cell receptor (TCR) Vβ chain with the major histocompatibility complex antigen, and induce T cell receptor signaling (TCR signaling). However, since the responding T cells are not selected by antigen specificity, a large number of T cells are activated simultaneously, leading to an excessive production of cytokines called "cytokine storm." When such cytokine storm is induced, the apoptosis of T cells and extensive differentiation of regulatory T cells occur to suppress excessive immune responses, which may exhaust the immune system of patients and may attenuate the immune functions for a long period of time in some cases (Non-Patent Document 2).

By the way, algae are conventionally utilized in food products, feeds, and so on. As one of the methods of utilizing algae, an antiviral agent comprising an extract of Coccomyxa algal body as an active ingredient has been disclosed (Patent Document 1). In addition, the development of an anti-influenza agent and a herpesvirus recurrence onset inhibitor, each comprising an extract of Coccomyxa algal body as an active ingredient, has also been promoted (Patent Documents 2 and 3). Moreover, Patent Document 4 describes that cytotoxic T cells having an antigen-specific cytotoxic activity are induced *in vitro* by incubating progenitor cells having an ability to differentiate into cytotoxic T cells with antigen-presenting cells in the presence of fucoidan, a sulfated polysaccharide derived from brown algae.

### Prior Art Documents

### Patent Documents

Patent Document 1: Japanese Patent No. 4411523
Patent Document 2: JP Patent Publication (Kokai) No. 2020-019730 A
Patent Document 3: JP Patent Publication (Kokai) No. 2020-019729 A
Patent Document 4: Japanese Patent No. 4231688

### Non-Patent Documents

Non-Patent Document 1: J. E. Alouf, et al., Int J Med Microbiol 292 (2003) 429-440.10.1078/1438-4221-00232.
Non-Patent Document 2: A. Taylor, et al., j Immunol 185 (2010) 6591-6598.

### Summary of Invention

### Objects to be Solved by the Invention

Regulatory T cells suppress excessive immune responses and play an important role in maintaining the homeostasis of living bodies. However, there has been a problematic case where the apoptosis of T cells and proliferation of regulatory T cells may suppress immune functions for a long period of time and the immune system in the body may not function normally. Therefore, it has been desired to develop a novel means for suppressing overactivation of T cells after stimulation of the T cells with superantigens or the like and the subsequent exhaustion of the immune system.

Hence, it is an object of the present invention to provide a means capable of appropriately regulating differentiation of activated T cells, in order to avoid overactivation of T cells after stimulation of the T cells with superantigens or the like and the subsequent decrease in the immune functions (exhaustion of the immune system).

### Means for Solving the Objects

As a result of intensive studies directed towards achieving the aforementioned object, it was found that an extract of the genus Coccomyxa microalgae is capable of accelerating differentiation of T cells into memory T cells, while promoting proliferation of activated T cells (effector T cells). Thus, it was suggested that an extract of the genus Coccomyxa microalgae suppresses overactivation of T cells and preserves memory T cells, so that attenuation of the immune functions may be prevented and decreased immune functions may be improved. According to the present invention, the following inventions are provided.

[1] A T cell differentiation-regulating agent, comprising, as an active ingredient, an algal body of microalgae belonging to the genus Coccomyxa, a dry powder thereof, or an extract thereof.
[2] The T cell differentiation-regulating agent according to [1], which has an action to promote proliferation of activated effector T cells, and an action to preserve memory T cells.
[3] The T cell differentiation-regulating agent according to [1] or [2], which suppresses overactivation of T cells.
[4] The T cell differentiation-regulating agent according to any one of [1] to [3], wherein the microalga is a Coccomyxa sp. KJ strain or a mutant strain thereof.
[5] The T cell differentiation-regulating agent according to any one of [1] to [4], which is a medicament, a quasi-drug, a cosmetic product, or a miscellaneous product.
[6] The T cell differentiation-regulating agent according to any one of [1] to [4], which is a food product, a food additive, or a feed.
[7] A composition, comprising the T cell differentiation-regulating agent according to any one of [1] to [6].
[8] The composition according to [7], which is a medicament, a quasi-drug, a cosmetic product, or a miscellaneous product.
[9] The composition according to [7], which is a food product, a food additive, or a feed.
[A] A method for regulating differentiation of T cells, comprising administering an algal body of microalgae belonging to the genus Coccomyxa, a dry powder thereof, or an extract thereof, to a subject who is in need of regulation of differentiation of T cells.
[B] An algal body of microalgae belonging to the genus Coccomyxa, a dry powder thereof, or an extract thereof T cells, which is for use in the treatment of regulating differentiation of T cells.
[C] Use of an algal body of microalgae belonging to the genus Coccomyxa, a dry powder thereof, or an extract thereof, for production of a T cell differentiation-regulating agent.

### Advantageous Effects of Invention

According to the present invention, there can be provided a T cell differentiation-regulating agent capable of appropriately regulating differentiation of T cells. Thereby, it is expected that overactivation of T cells after stimulation of the T cells with superantigens or the like and the subsequent decrease in the immune functions (exhaustion of the immune system) will be suppressed.

### Brief Description of Drawings

[Fig. 1] Fig. 1 shows a total cell count in the case of stimulating human PBMCs with TSST-1 in the presence of various concentrations of CE (Student's t-test: *p = 0.05, **p = 0.01, *** p = 0.001, **** p = 0.0001).
[Fig. 2] Fig. 2 shows the ratio between the cells in the small lymphocyte gate (upper view) and the cells in the large lymphocyte gate (activated lymphocytes) (lower view), among a total cell count in the case of stimulating human PBMCs with TSST-1 in the presence of various concentrations of CE.
[Fig. 3] Fig. 3 shows morphological observation results (BZ-X710 Phase Difference, KEYENCE) (right panels) in the case of stimulating human PBMCs with TSST-1 in the presence of various concentrations of CE. The left panels show the FCM patterns (FSC/SSC).
[Fig. 4] Fig. 4 shows the FCM patterns of lymphocyte subsets.
[Fig. 5] Fig. 5 shows the percentage of lymphocyte subsets (T cells, Th cells, Tc cells, B cells, NK cells and NKT cells) after the cells are stimulated with TSST-1 and are then cultured for 72 hours in the presence of different concentrations of CE (Student's t-test: *p = 0.05, ** p = 0.01, *** p = 0.001). The ratio between the cells in the small lymphocyte gate (black bar) and the cells in the large lymphocyte gate (white bar) is shown.
[Fig. 6] Fig. 6 shows the results of analyzing proliferation of T cells stimulated with anti-CD3 mAb or anti-CD3/CD28 microbeads. The horizontal axis indicates the fluorescence intensity of CFSE, and the peak indicates cell division (left 9 panels: 24 hours; middle 9 panels: 48 hours; right 9 panels: 72 hours). The upper panels show the results without TSST-1 stimulation, the middle panels show the results with TSST-1 stimulation, and the lower panels show the results with TSST-1 stimulation and 3000 µg/mL CE.
[Fig. 7] Fig. 7 shows the mean fluorescence intensity (MFI) of CD25 in the CD4 gate (Th cells) and the CD8 gate (Tc cells). The black bar indicates the results of the small lymphocyte gate, and the white bar indicates the results of the large lymphocyte gate.
[Fig. 8] Fig. 8 shows the mean fluorescence intensity (MFI) of PD-1 in the CD4 gate (Th cells) and the CD8 gate (Tc cells) (Student's t-test: *p = 0.05, ** p = 0.01, *** p = 0.001). The black bar indicates the results of the small lymphocyte gate, and the white bar indicates the results of the large lymphocyte gate.

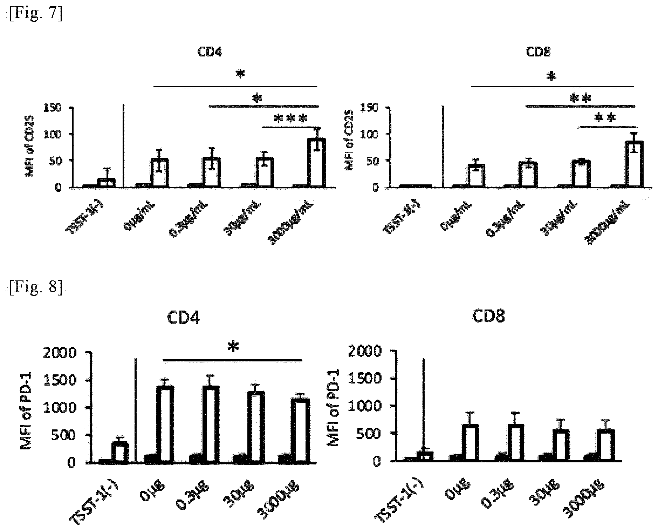

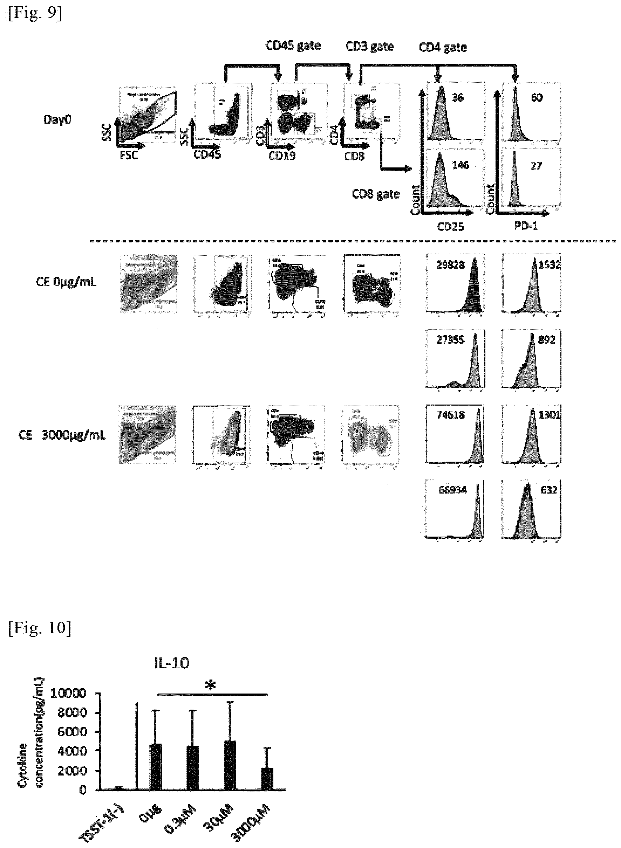

[Fig. 9] Fig. 9 shows the FCM patterns of activated T cells and exhausted T cells in the presence or absence of CE. The panels above the dotted line show a typical FACS patterns of pre-cultured (Day 0) T cells. In Fig. 9, small lymphocytes and large lymphocytes are gated into CD45+CD3+ cells, CD4T cells are divided from CD8T cells, and the expression levels of CD25 and PD-1 are analyzed. Regarding Day 3 (the panels below the dotted line), only the cells in the large lymphocyte gate are shown. Besides, the analysis results of both the small lymphocyte gate and the large lymphocyte gate are shown in Figs. 7 and 8.
[Fig. 10] Fig. 10 shows the concentration of IL-10 in the culture supernatant.
[Fig. 11] Fig. 11 shows the percentage of Th1, Th2, and Th17 cells before culture (Day 0), among CD4+ T cells. The black bar indicates the results of the small lymphocyte gate, and the white bar indicates the results of the large lymphocyte gate.
[Fig. 12] Fig. 12 shows the percentage of Th1, Th2, and Th17 cells, after different concentrations of CE are added and the mixture is then cultured for 72 hours. The black bar indicates the results of the small lymphocyte gate, and the white bar indicates the results of the large lymphocyte gate.
[Fig. 13] Fig. 13 shows the FCM patterns of Th cell subsets in the presence or absence of CE. The panels above the dotted line show a typical FACS patterns of pre-cultured (Day 0) T cells. In Fig. 13, small lymphocytes and large lymphocytes are gated into CD45+CD3+ cells, and the Th cell subsets are analyzed according to the expression of a chemokine receptor (Day 0). Regarding Day 3 (the panels below the dotted line), only the cells in the small lymphocyte gate are shown.
[Fig. 14] Fig. 14 shows the concentrations of individual cytokines produced by human PBMCs. The longitudinal axis indicates the concentrations of individual cytokines (pg/ml) (Student's t-test: *p = 0.05, ** p = 0.01).
[Fig. 15] Fig. 15 shows the expression of naive/memory T cell markers that are unstimulated (TSST-1(-)) or are stimulated with 0 to 3000 µg/ml CE, and shows the FCM patterns of CD45RA and CD62L expression on T cells.
[Fig. 16] Fig. 16 shows the percentage of CD45RA/CD62L double positive cells in the analysis of Fig. 15 (Student's t-test: ** p = 0.01).
[Fig. 17] Fig. 17 shows the analysis results of the expression levels of CCR7, CD127, CXCR3, CD95 and CD45RO. The five data in each panel indicate, from the top, unstimulated (TSST-1(-)), CE concentrations of 0 µg/ml , 0.3 µg/ml , 30 µg/ml and 3000 µg/ml.
[Fig. 18] Fig. 18 shows the FCM patterns for analyzing the expression levels of CD45RA and CD45RO. In Fig. 18, small lymphocytes or large lymphocytes are gated into CD3+ cells, CD4T cells are divided from CD8T cells, and the expression levels of CD45RA and CD45RO are analyzed.

### Embodiments of Carrying out the Invention

Hereinafter, the present invention will be described in detail. The following explanations of constituent features may be made based on representative embodiments or specific examples. However, the present invention is not limited to such embodiments.

### 1.T cell differentiation-regulating agent

In the present description, the T cell differentiation-regulating agent is an agent for regulating differentiation of T cells associated with activation thereof. Specifically, the T cell differentiation-regulating agent of the present invention promotes proliferation of already activated T cells (effector T cells), and at the same time, preserves an inactivated cell population (memory T cells), so that depletion of T cells is suppressed. Thus, since depletion of T cells is suppressed, it becomes possible to prevent a patient from becoming an immunosuppressed state (immune function-decreased state) or to allow a patient to escape from such an immunosuppressed state (immune function-decreased state), and thus, the improvement of the immune functions is expected. Besides, since T_{SCM} cells among memory T cells (T_{SCM} cells, T_{CM} cells, and T_{EM} cells) have high self-replication ability and high proliferative ability and survive for a long period of time, an increase in the ratio of the T_{SCM} cells can effectively suppress exhaustion of T cells and can preserve the T cells.

Moreover, the T cell differentiation-regulating agent of the present invention regulates differentiation of activated T cells (effector T cells), reduces the ratio of exhausted T cells, and suppresses induction of apoptosis. Thus, the present T cell differentiation-regulating agent promotes proliferation of the activated T cells (effector T cells), but acts to prevent exhaustion of T cells. If the ratio of the exhausted T cells and apoptosis can be reduced, excessive suppression of the immune functions can be prevented.

Furthermore, the T cell differentiation-regulating agent of the present invention regulates production of inflammatory cytokines. Since overproduction of inflammatory cytokines leads to induction of cytokine storms, it is expected that regulation of production of inflammatory cytokines will exhibit the effect of suppressing the occurrence of cytokine storms and the relevant attenuation of immune functions.

The term "agent" is used in the present description to mean that the agent may only consist of the "algal body of microalgae belonging to the genus Coccomyxa, a dry powder thereof, or an extract thereof" serving as an active ingredient, or may also comprise additives other than the above-described active ingredient. That is to say, the T cell differentiation-regulating agent of the present invention also includes the "algal body of microalgae belonging to the genus Coccomyxa, a dry powder thereof, or an extract thereof" itself, which is for use in regulation of differentiation of T cells.

### 2. Active ingredient of T cell differentiation-regulating agent

The T cell differentiation-regulating agent of the present invention comprises, as an active ingredient, an algal body of microalgae belonging to the genus Coccomyxa, a dry powder thereof, or an extract thereof.

The microalga of the genus Coccomyxa is not particularly limited. A preferred example may be a Coccomyxa sp. KJ strain or a mutant strain thereof. The Coccomyxa sp. KJ strain (KJ DENSO) was deposited with International Patent Organism Depositary, Biotechnology Center, National Institute of Technology and Evaluation (NITE-IPOD) (Room 120, 2-5-8 Kazusa-Kamatari, Kisarazu-shi, Chiba-ken, Japan) under Accession No. FERM P-22254 on June 4, 2013, and was then transferred to an international deposition under the provisions of the Budapest Treaty on June 2, 2015, and received Accession No. FERM BP-22254.

The mutant strain of the Coccomyxa sp. KJ strain can be obtained by irradiation with ultraviolet ray, X-ray, γ-ray, etc., a mutagen treatment, heavy beam irradiation, genetic manipulation (e.g. introduction of foreign genes, gene disruption, genetic modification by genome editing, etc.), and the like. As long as a mutant strain capable of regulating differentiation of activated T cells can be obtained, the method of obtaining such a mutant strain, characteristics thereof, and the like are not particularly limited.

The method of culturing the genus Coccomyxa microalgae is not particularly limited. As a medium for culturing the genus Coccomyxa microalgae, a medium that is commonly used for the culture of microalgae may be used, and for example, either a known medium for freshwater microalgae or a known medium for marine microalgae, each of which contains various types of nutrient salts, trace metal salts, vitamins, etc., can be used. As such a medium, for example, an AF6 medium can be used. The composition of the AF6 medium (per 100 ml) is as follows.

| | |
|---|---|
| NaNO₃ | 14 mg |
| NH₄NO₃ | 2.2 mg |
| MgSO₄·7H₂O | 3 mg |
| KH₂PO₄ | 1 mg |
| K₂HPO₄ | 0.5 mg |
| CaCl₂·2H₂O | 1 mg |
| CaCO₃ | 1 mg |
| Fe-citrate | 0.2 mg |
| Citric acid | 0.2 mg |
| Biotin | 0.2 µg |
| Thiamine HCl | 1 µg |
| Vitamin B₆ | 0.1 µg |
| Vitamin B₁₂ | 0.1 µg |
| Trace metals | 0.5 mL |
| Distilled water | 99.5 mL |

Examples of the nutrient salts may include: nitrogen sources such as NaNO₃, KNO₃, NH₄Cl, and urea; and phosphorus sources such as K₂HPO₄, KH₂PO₄, and sodium glycerophosphate. Examples of the trace metals may include iron, magnesium, manganese, calcium, and zinc. Examples of the vitamins may include vitamin B₁ and vitamin B₁₂.

As a culture method, stirring may be carried out with carbon dioxide supply under ventilation conditions. Upon the culture, the cells are cultured under light irradiation with light/dark cycles, such as light irradiation with a fluorescent light for 12 hours and dark conditions for 12 hours, or under continuous light irradiation. The culture conditions are not particularly limited, either, as long as they do not adversely affect proliferation of the genus Coccomyxa microalgae. For example, the pH of the culture solution is preferably set to be pH 3 to 9, and the culture temperature is preferably set to be 10°C to 35°C.

With regard to the method of culturing the Coccomyxa sp. KJ strain, the methods described in JP Patent Publication (Kokai) No. 2015-15918 A, International Publication WO2015/190116, Satoh, A. et al., Characterization of the Lipid Accumulation in a New Microalgal Species, Pseudochoricystis ellipsoidea (Trebouxiophyceae) J. Jpn. Inst. Energy (2010) 89: 909-913., etc. can be adopted, as appropriate.

Dry powders of the algal body can be prepared by subjecting the recovered algal body to a dry treatment and a crushing (pulverization) treatment. As a dry treatment, for example, drum drying, spray drying, freeze drying, etc. can be adopted. For a crushing treatment, a bead-type crusher, a homogenizer, a French press, a mixer/blender, a fine grinder, etc. can be utilized. The order of performing the dry treatment and the crushing treatment is not limited. The dry treatment and the crushing treatment may also be performed simultaneously by utilizing an apparatus equipped with both of the drying and crushing functions.

The particle diameter of the dry powders is not particularly limited. For example, dry powders having a mean particle diameter of 0.2 µm to 2 mm, and preferably 0.4 µm to 400 µm, are preferable.

The extract of the Coccomyxa sp. KJ strain is a substance extracted from the dry powders of the Coccomyxa sp. KJ strain, using water as a solvent. The recovered algal body is dried, and the dried matter is then suspended and mixed in distilled water, followed by centrifugation. The obtained culture supernatant is collected to obtain the extract of the Coccomyxa sp. KJ strain. The collected supernatant may be directly used as an extract of the algal body. Otherwise, the collected supernatant may be subjected to a dry treatment, and the obtained product may be used as an extract. Besides, as a dry treatment of the supernatant, for example, drum drying, spray drying, freeze drying, etc. can be adopted. The temperature of water used for the extraction may be a high temperature of 85°C or may be a low temperature of 10°C or lower. The temperature of the water is not particularly limited, as long as it is within a range in which the active ingredient can be sufficiently extracted. The temperature of the water is preferably in the range of 10°C to 50°C. The extraction time is preferably set to be 1 to 100 hours. As dry powders used for the extraction, a residue obtained by extracting fat-soluble components, using an organic solvent such as acetone, ethanol or hexane, can be used. In addition, the microalgae used are not limited to the Coccomyxa sp. KJ strain, and another genus Coccomyxa may also be used.

### 3. Intended use and/or usage of T cell differentiation-regulating agent

The T cell differentiation-regulating agent of the present invention and a composition comprising the same can be used in various intended uses. The T cell differentiation-regulating agent of the present invention and a composition comprising the same can be used, for example, as medicaments (therapeutic agents or preventive agents), food products, or feeds.

### (1) Medicament, quasi-drug, cosmetic product and miscellaneous product

The T cell differentiation-regulating agent of the present invention or a composition comprising the same can be used, for example, as a medicament, a quasi-drug, a cosmetic product, a miscellaneous product, a food product, a food additive, or a feed, and can be used, for example, in the treatment for the improvement of immune functions or prevention of immune attenuation. The effects of the medicament include (1) prevention of an immunosuppressed state (immune function-decreased state), (2) the improvement of immune functions, (3) suppression of excessive immune responses, and the like.

Formulation for producing a medicament, a quasi-drug, a cosmetic product, and a miscellaneous product can be carried out according to ordinary methods. Using pharmaceutically acceptable additives (for example, an excipient, a disintegrant, a buffer, an emulsifier, a suspending agent, a soothing agent, a stabilizer, a preservative, an antiseptic, a normal saline, etc.), formulation may be carried out.

Examples of the excipient that can be used herein may include lactose, starch, sorbitol, D-mannitol, and white sugar.

Examples of the disintegrant that can be used herein may include starch, carboxymethyl cellulose, and calcium carbonate.

Examples of the buffer that can be used herein may include phosphate, citrate, and acetate.

Examples of the emulsifier that can be used herein may include gum Arabic, sodium alginate, and tragacanth.

Examples of the suspending agent that can be used herein may include glycerin monostearate, aluminum monostearate, methyl cellulose, carboxymethyl cellulose, hydroxymethyl cellulose, and sodium lauryl sulfate.

Examples of the soothing agent that can be used herein may include benzyl alcohol, chlorobutanol, and sorbitol.

Examples of the stabilizer that can be used herein may include propylene glycol and ascorbic acid.

Examples of the preservative that can be used herein may include phenol, benzalkonium chloride, benzyl alcohol, chlorobutanol, and methylparaben.

Examples of the antiseptic that can be used herein may include benzalkonium chloride, paraoxybenzoic acid, and chlorobutanol.

The dosage form to be formulated is not particularly limited.

Examples of the dosage form may include a tablet, a powder agent, a fine granule, a granule, a capsule, syrup, an injection, an external agent (ointment, cream, lotion, a liquid agent, a gelling agent, a patch, a plaster, a tape, an aerosol agent, etc.), and a suppository.

The medicament and the quasi-drug are applied to a subject by oral administration or parenteral administration (local injection into the affected area, intravenous, intraarterial, subcutaneous, intradermal, intramuscular or intraperitoneal injection, transdermal, intranasal or transmucosal administration, etc.) according to their dosage forms. Moreover, systemic administration and local administration are also applied depending on the subject. These administration routes are not mutually exclusive, and optionally selected two or more administration routes can also be used in combination.

The medicament, quasi-drug or cosmetic product of the present invention comprises the active ingredient in an amount necessary to obtain the expected effects (i.e., in a therapeutically or preventively effective amount). The amount of the active ingredient comprised in the medicament of the present invention is generally different depending on the dosage form, and the amount of the active ingredient is set, for example, within the range of about 0.1% by weight to about 99% by weight, in order to achieve the desired applied dose.

The applied dose of the medicament, quasi-drug or cosmetic product of the present invention is set to obtain the expected effects. Upon setting a therapeutically or preventively effective dose, the symptoms, age, gender, and body weight of the patient are generally taken into consideration. A person skilled in the art can set an appropriate applied dose, while taking these factors into consideration. As an example, the applied dose for an adult subject (body weight: about 60 kg) can be set to be 1 mg to 20 mg of the active ingredient per day, and preferably 2 mg to 10 mg of the active ingredient per day. The administration schedule can be, for example, from once a day to several times a day, once every two days, or once every three days. When the administration schedule is prepared, the patient's conditions, the duration of the effects of the active ingredient, and the like can be taken into consideration.

In parallel with the treatment or prevention with the medicament of the present invention, a treatment with other medicaments (e.g., T cell differentiation-regulating agents such as glucocorticoids) may also be applied. If an agent having an action mechanism different from that of the active ingredient of the present invention is used in combination with the present medicament, combined action/effects can be obtained, and when such combined use is applied to the improvement of immune functions, the therapeutic effects can be increased.

The present invention also provides a method for performing a treatment for improving immune functions or for preventing a decrease in immune functions, which is characterized in that the method comprises administering a therapeutically or preventively effective amount of medicament comprising the T cell differentiation-regulating agent of the present invention, to a patient who is affected with or is likely to be affected with a decrease in immune functions. The subject of the treatment or prevention is typically a human, but the treatment or prevention may also be applied to mammals other than humans (e.g., a monkey, a bovine, a swine, a horse, a goat, sheep, a dog, a cat, a rabbit, etc.), birds (a chicken, a geese, etc.), and the like.

### (2) Food product, food additive, and feed

Examples of a food product comprising the T cell differentiation-regulating agent may include general food products (cereals, vegetables, meats, various types of processed foods, sweets and/or desserts, milk, soft drinks, fruit juice drinks, coffee drinks, vegetable juice drinks, alcoholic drinks, etc.), nutritional supplements (supplements, nutritional drinks, etc.), food additives, foods for companion animals, and nutritional supplements for companion animals. Nutritional supplements and food additives can be provided in the form of powders, granules, tablets, pastes, liquids, etc. By providing the present T cell differentiation-regulating agent in the form of a food product composition, it becomes easy to take the active ingredient of the present invention on a daily basis or on a continuous basis.

In addition, the T cell differentiation-regulating agent of the present invention and the composition can also be used as food additives.

Examples of the feed comprising the T cell differentiation-regulating agent may include feeds (feeds for livestock, poultries, etc.) and pet foods.

The food product or feed of the present invention preferably comprises the active ingredient in an amount that is expected to exhibit therapeutic or preventive effects. The amount to be added can be determined, while taking into consideration the conditions, age, gender, body weight, etc. of a subject to whom the food or feed is to be used.

The characteristics of the present invention will be more specifically described in the examples and comparative examples described below. In the following examples, the materials used, the amounts, ratios, the contents of the treatments, the treatment procedures, etc. may be modified, as appropriate, unless they overstep the spirit and the scope of the invention. Accordingly, the invention should not be restrictively interpreted by the following specific examples.

### Examples

### < Methods >

### 1. Preparation of extract of genus Coccomyxa microalgae

Freeze-dried Coccomyxa sp. KJ (IPOD FERM BP-22254) (2.5 g) was added to 25 mL of distilled water, followed by shaking and mixing them at 37°C for 6 hours. Subsequently, the obtained suspension was centrifuged at 6000 rpm for 10 minutes. The supernatant was collected and was then freeze-dried.

### 2. Culture of human PBMCs

Human PBMCs were isolated from peripheral blood (PB), and the isolated cells were then washed with a phosphate buffered saline (PBS) for 5 minutes at 4°C at 300 x g, and were then seeded on a 6-well plate. Thereafter, the cells were cultured in the RPMI 1640 medium "Nissui" (manufactured by Nissui Pharmaceutical Co., Ltd.) (1 × 10⁶/ml) under conditions of 37°C and 5% CO₂, in the presence of 1 µg/ml TSST-1 (Toxin Tec. Sarasota, USA). It is to be noted that the RPMI 1640 medium "Nissui" comprised 10% fetal calf serum (FCS, manufactured by Sigma-Aldrich) and antibiotics (0.1 mg/ml streptomycin and 100 units/ml penicillin (Meiji Seika Kaisha, Ltd.)). To the thus cultured cells, a different amount of the extract (CE) of the genus Coccomyxa microalgae was added, and the cells were then harvested 24 hours, 48 hours, and 72 hours after the culture, followed by washing the harvested cells with a phosphate buffered saline (PBS).

### 3. Analysis by flow cytometry

For the sorting of T cells, Pan T Cell Isolation Kit (manufactured by Miltenyi Biotec) was used. The human PBMCs harvested and washed by the aforementioned method were incubated with Pan T Cell Biotin-Antibody Cocktail at 4°C for 5 minutes. Subsequently, 40 µl of a washing buffer was added, and 20 µl of Pan T Cell MicroBead Cocktail were added, and the obtained mixture was then incubated at 4°C for 10 minutes. T cells were sorted by Automax Aystem (program: depletion) (manufactured by Miltenyi Biotec) and were then labeled by CellTrace^{™} Cell Proliferation Kit (manufactured by Thermo Fisher Scientific) according to the product manuals. Specifically, 5-(6)-carboxyfluorescein-diacetate-succinimidyl ester (CFSE) powders were mixed with dimethyl sulfoxide (DMSO, manufactured by FUJIFII,M WAKO PURE CHEMICAL CORPORATION) to a concentration of 5 mM, and the T cells were then incubated with the CFSE (final concentration: 5 µM) at 37°C in a dark place for 20 minutes. Thereafter, the T cells were washed with PBS, and were then resuspended in an RPMI 1640 medium. After that, the T cells were stimulated with Dynabeads Human T-Activator CD3/CD28 (manufactured by Thermo Fisher Scientific) or anti-CD3 coated on a microplate. The T cells were harvested over time for 3 days, and the cell cycle was analyzed by FCM as follows.

Mononuclear cells were harvested from each well, were then counted, and were then stained with an appropriate diluted solution of fluorescent dye-labeled mAb for 15 minutes (in 4°C environment). Thereafter, the cells were washed with 1% (w/v) bovine serum albumin (BSA, manufactured by Sigma-Aldrich) containing PBS. The resulting cells were analyzed at 72 hours, and the surface expression of differentiation antigens was detected using BD LSRFortessa^{™} Flow Cytometer (manufactured by BD Biosciences). In each analysis, propidium iodide (PI)-negative cells were further gated to extract living cells, which were then further analyzed with FlowJo software v10.3 (BD). The mAbs used for staining are as follows. For the CFSE analysis, BD FACSVerse^{™} Flow Cytometer (manufactured by BD Biosciences) was used.

**[Table 1]**

| Antibody | Clone | Company | Cat. No. |
|---|---|---|---|
| FITC anti-human CD3 | UCHT1 | BioLegend | 300406 |
| APC anti-human CD3 | SK7 | BD Bioscience | 340440 |
| APC/Cy7 anti-human CD3 | SK7 | BioLegend | 344818 |
| APC anti-human CD4 | RPA-T4 | BioLegend | 300514 |
| BrilliantViolet510^{™} anti-human CD4 | OKT4 | BioLegend | 317444 |
| Alexa Fluor^{®}700 anti-human CD4 | OKT4 | eBioscience | 317426 |
| Alexa Fluor^{®}700 anti-human CD8 | HIT8a | BioLegend | 300920 |
| BV395 anti-human CD8 | RPA-T8 | Biosciences | 563795 |
| PE anti-human CD14 | 63D3 | BioLegend | 367103 |
| BrilliantViolet650^{™} anti-human CD16 | 3G8 | BioLegend | 302041 |
| APC/Cy7 anti-human CD19 | HIB19 | BioLegend | 302218 |
| PE anti-human CD25 | BC96 | BioLegend | 302606 |
| PE/Cy7 anti-human CD25 | BC96 | BioLegend | 302612 |
| APC anti-human CD33 | P67.6 | BioLegend | 366605 |
| PE/Cy7 anti-human CD40L | 24-31 | BioLegend | 310832 |
| FITC anti-human CD44 | BJ18 | BioLegend | 338804 |
| Pacific Blue^{™} anti-human CD45 | HI30 | BioLegend | 304022 |
| BUV737 anti-human CD45 | HI30 | Biosciences | 748719 |
| BrilliantViolet650^{™} anti-human CD45RA | HI100 | BioLegend | 304136 |
| APC/Cy7 anti-human CD45RO | UCHL1 | BioLegend | 304228 |
| PE/Cy7 anti-human CD56 | NCAM16.2 | BD Bioscience | 335791 |
| PE anti-human CD62L | DREG-56 | BioLegend | 304806 |
| BV421 anti-human CD95 | DX2 | BioLegend | 305624 |
| PE/Cy7 anti-human CD127 | A01905 | BioLegend | 351320 |
| BrilliantViolet510^{™} anti-human CD127 (IL-7Rα) | A019D5 | BioLegend | 351332 |
| PerCP/Cy5.5 anti-human CD183 (CXCR3) | G025H7 | BioLegend | 353714 |
| BrilliantViolet421^{™} anti-human CD185 (CXCRS) | J252D4 | BioLegend | 356920 |
| APC/Cy7 anti-human CD185 (CXCR5) | J252D4 | BioLegend | 356926 |
| BrilliantViolet421^{™} anti-human CD194 (CCR4) | L291H4 | BioLegend | 359414 |
| BrilliantViolet510^{™} anti-human CD196 (CCR6) | G034E3 | BioLegend | 353424 |
| APC anti-human CD197 (CCR7) | G043H7 | BioLegend | 353214 |
| BUV395 anti-human CD274 (PD-L1) | MIH1 | BD Bioscience | 740320 |
| Alexa Fluor^{®}700 anti-human CD278 (ICOS) | C398.4A | BioLegend | 313528 |
| PE anti-human CD279 (PD-1) | EH12.2H7 | BioLegend | 329906 |
| PerCP/Cy5.5 anti-human CD279 (PD-1) | EH12.2H7 | BioLegend | 329914 |
| FITC anti-human TCRα/β | IP26 | BioLegend | 306706 |
| PI | | SIGMA | P4170 |

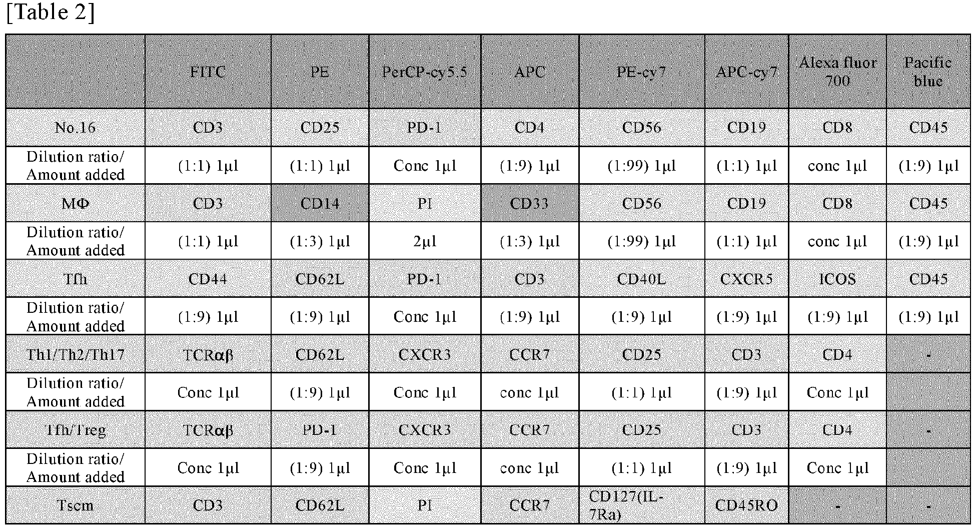

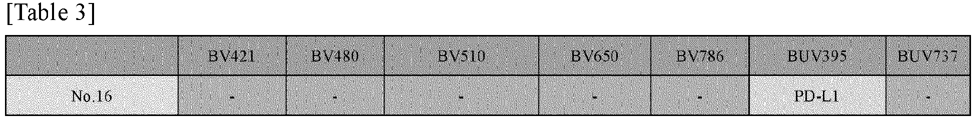

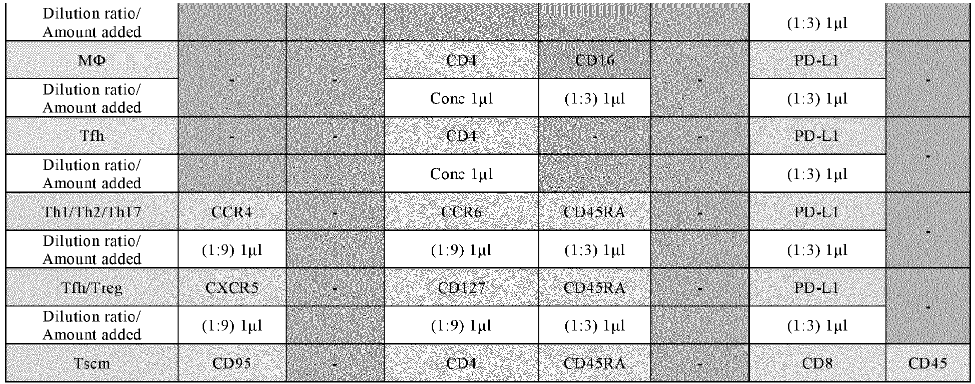

### 4. Quantification of cytokines

The culture supernatant obtained in < 2. Culture of human PBMCs > was harvested, and cytokines were then quantified using Bead-Based Multiplex LEGENDplex (manufactured by BioLegend) according to the product manuals. Specifically, 25 µL of the supernatant and 25 µL of Capture Beads were mixed with each other, and the obtained mixture was then incubated at room temperature for 2 hours. Thereafter, the beads were washed and were then mixed with a detection antibody, and the obtained mixture was then incubated at room temperature for 1 hour. Subsequently, SA-PE was added to the reaction mixture, and the thus obtained mixture was then incubated at room temperature for 30 minutes. Finally, the resulting beads were washed and were then subjected to flow cytometry. The quantified cytokines were IL-1α, IFN-α, IFN-γ, TNF-α, MCP-1, IL-2, IL-4, IL-5, IL-6, IL-8, IL-9, IL-10, IL-12p70, IL-13, IL-17A, IL-17F, IL-18, IL-22, IL-23 and IL-33. For the analysis, BD FACSVerse^{™} Flow Cytometer (manufactured by BD Biosciences) was used. Data were analyzed by LEGENDPlex^{™} V8.0 software (manufactured by BioLegend), and the results were expressed in pg/ml, and were input into a database.

### 5. Statistical analysis

Statistical analysis was carried out using Microsoft Excel or ANOVA (Microsoft, Redmond, WA USA). Data were expressed as a mean value ± standard deviation.

### < Results >

### 1. CE regulates the number of activated T cells.

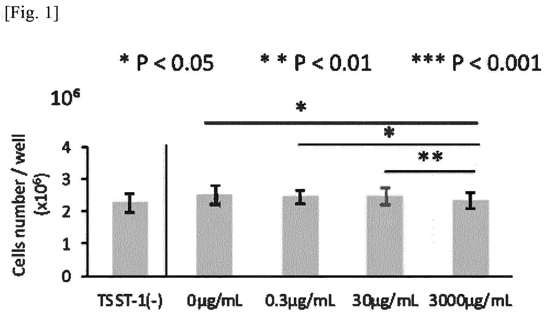

Fig. 1 shows a cell count in the case of stimulating human PBMCs with TSST-1 in the presence of various concentrations of CE. As shown in Fig. 1, total cell number was increased after the TSST-1 stimulation, while the level was significantly decreased in the CE concentration dependent manner.

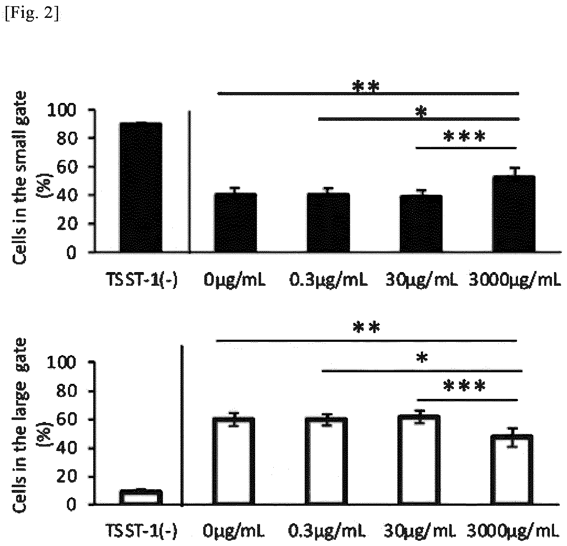

Fig. 2. shows the ratio between the cells in the large lymphocyte gate (activated lymphocytes) and the cells in the small lymphocyte gate. As shown in Fig. 2, the ratio of the T cells in the large lymphocyte gate, which indicates activation of lymphocytes, was decreased after stimulation with TSST-1 in a CE concentration-dependent manner.

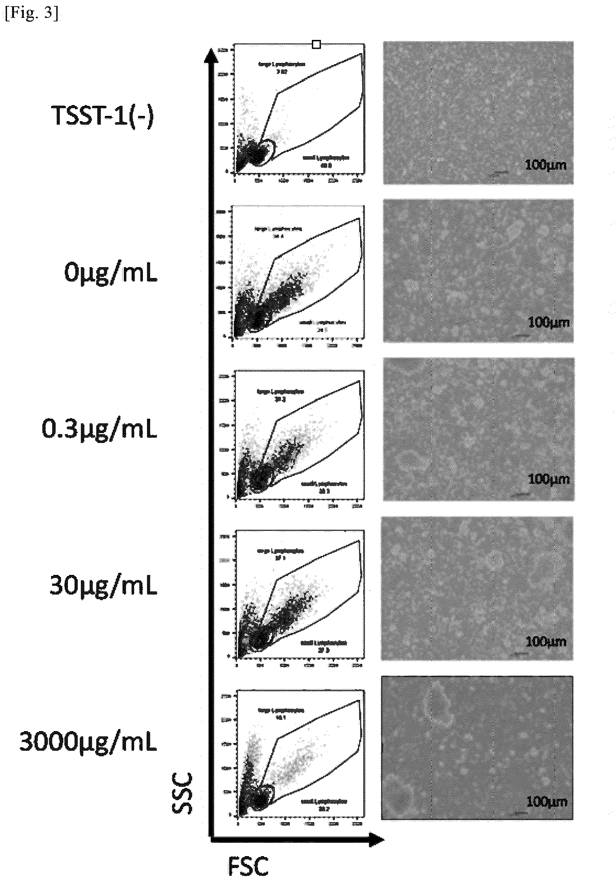

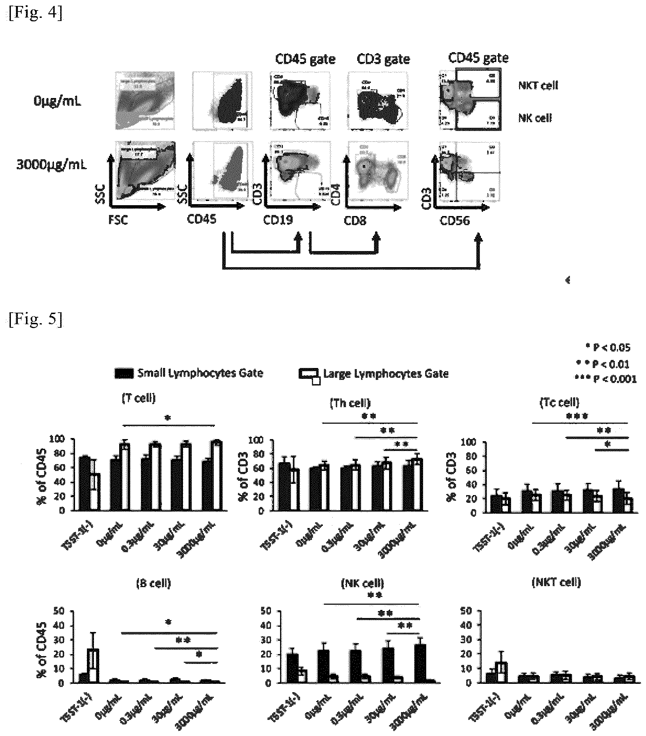

In addition, as shown in Fig. 3, the size of a cluster formed by aggregation of the cells was increased by 3000 µg/ml CE culture. Besides, the percentage of helper T cells (Th cells) was small, but was significantly increased. The percentage of killer T cells (Tc cells), B cells and NK cells was significantly decreased. The percentage of NKT cells was not changed (Fig. 4 and Fig. 5). These results suggest that CE affects activation of T cells after TSST-1 stimulation.

### 2. CE regulated proliferation of T cells.

Since the above-described results suggested that CE decreases the number of PBMCs and changes the form of aggregation, whether CE influences on the proliferation speed of T cells was examined.

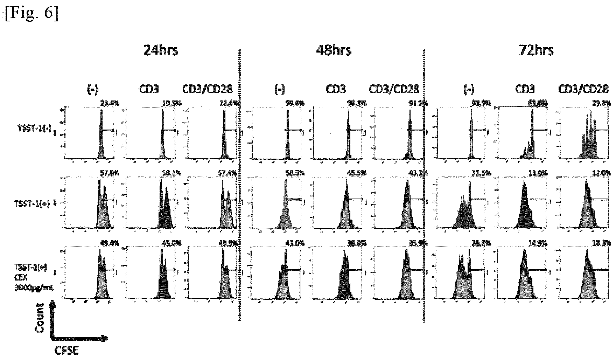

That is, T cells were stimulated with TSST-1 for 72 hours in the presence or absence of 3,000 µg/ml CE, and were then purified. Thereafter, the T cells were labeled with CFSE, and were then stimulated with anti-CD3 mAb alone or with anti-CD3- and anti-CD28mAb-conjugated beads. The cells were harvested 24 to 72 hours after the stimulation, and the degree of proliferation was measured according to a CFSE attenuation method. As a result, the T cells in the small lymphocyte gate and the cells in the large lymphocyte gate showed different levels of CFSE intensity, and this tendency remained even after 24 hours (Fig. 6). However, 48 hours after the stimulation, no high intensity peaks were observed, suggesting that the cells in the large lymphocyte gate had divided and the peaks had been decreased. Moreover, as shown in Fig. 6, the cells cultured in the presence of 3,000 µg/ml CE showed an early (48 hrs) initiation of proliferation, compared with cells cultured without CE; however, compared with T cells stimulated with TSST-1 alone, a considerable amount of the T cells remained in a non-proliferative state even on Day 3 (72 hrs). Among the TSST-1-stimulated cell fractions, CD3/CD28 stimulated cells showed less proliferation than CD3 stimulated cells, regardless of addition of CE.

These results suggested that, while CE preserves cells that are not proliferated by T cell stimulation and maintains the proliferative ability of proliferating cells, the CE accelerates proliferation of activated T cells.

### 3. CE enhances differentiation of effector T cells.

The above-described results suggested that activated T cells (effector T cells) proliferate more quickly, and that the effector T cells are produced more extensively by CE. Thus, the surface activation markers of the activated T cells (effector T cells) were analyzed.

In the large lymphocyte gate, the expression of CD25 and programmed cell death-1 (PD-1) in both CD4 and CD8 T cells was increased after TSST-1 stimulation. In the presence of CE, the CD25 expression was significantly increased in both CD4 and CD8 T cells, but the PD-1 expression was decreased particularly in CD4 T cells (Fig. 7, Fig. 8, and Fig. 9).

CD25 and PD-1 are not only activation markers, but also activation markers of regulatory T cells (Treg). Therefore, the secretion amount of IL-10, which has been known to be secreted from Treg cells and to suppress immune activation, was examined. As a result, IL-10 secretion was significantly decreased (Fig. 10), suggesting that CE did not enhance Treg differentiation, but that the cells were activated and were not exhausted.

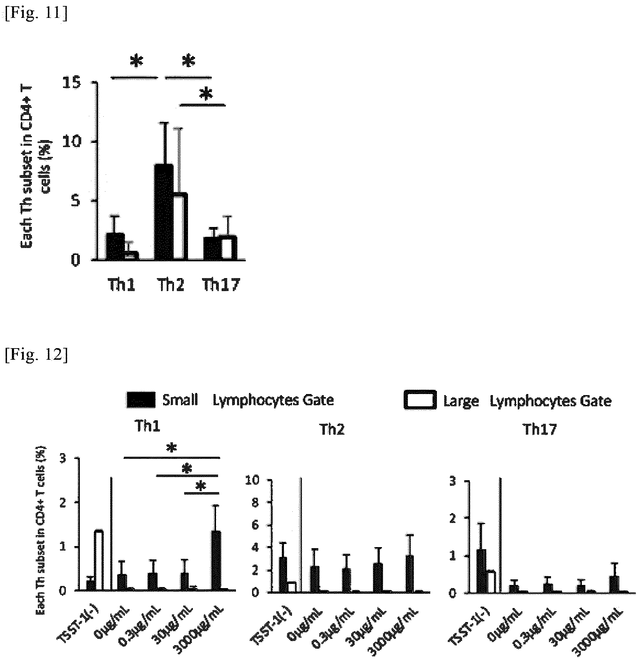

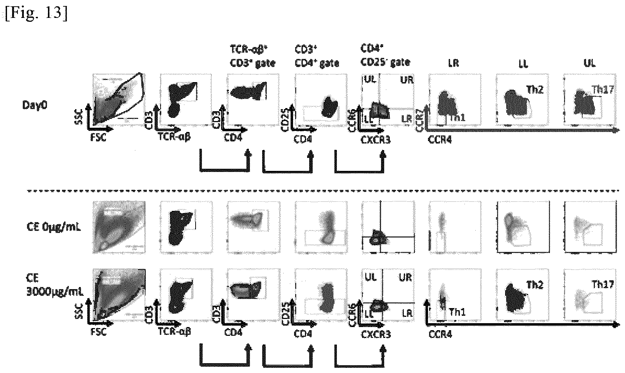

Furthermore, surface markers for detecting Th1, Th2, and Th17 cells were also analyzed. As a result, Th1 cells were significantly increased in the small lymphocyte gate in a CE concentration-dependent manner. Th2 and Th17 cells also tended to be increased (Fig. 11, Fig. 12, and Fig. 13).

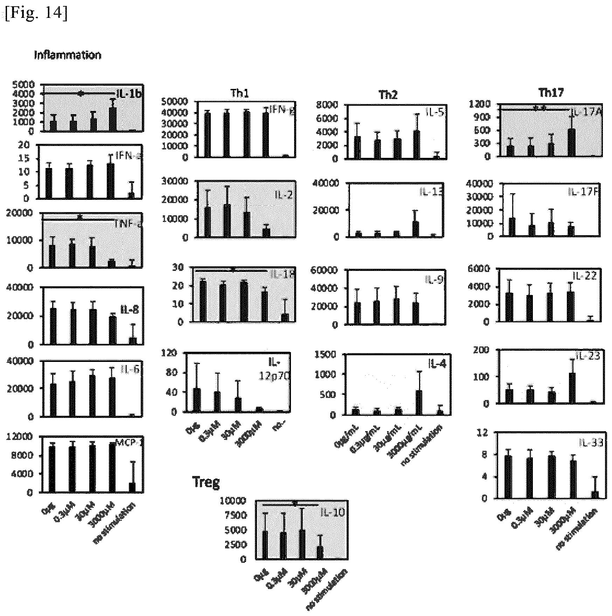

Further, the influence of CE upon secretion of cytokines was also analyzed. As a result, most of the inflammatory cytokines were maintained at low levels, but IL-1 and IL-17 were significantly increased, and IL-4 and IL-13 tended to be increased (Fig. 14). On the other hand, TNF-α, IL-18 and IL-2 were significantly decreased and IL-12p70 tended to be decreased. These results suggest that CE effectively developed effector T cells. Th1 cytokine tended to be decreased, but the ratio of Th1 cells was increased along with Th17 and Th2.

### 4. CE enhances differentiation of memory T cells.

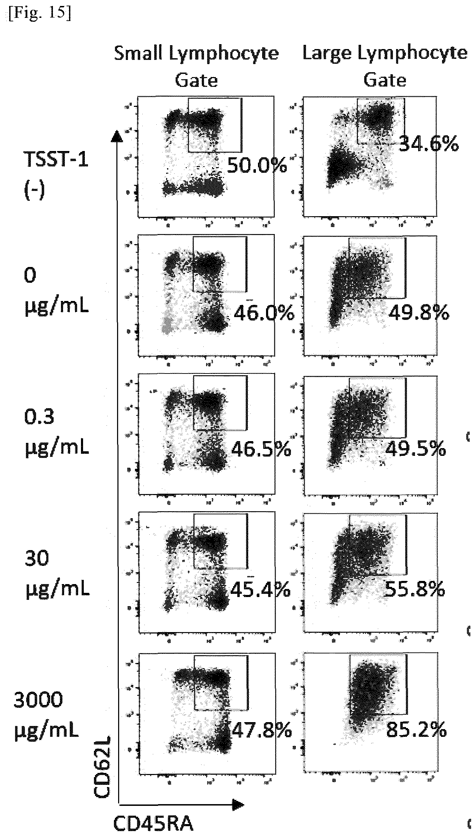

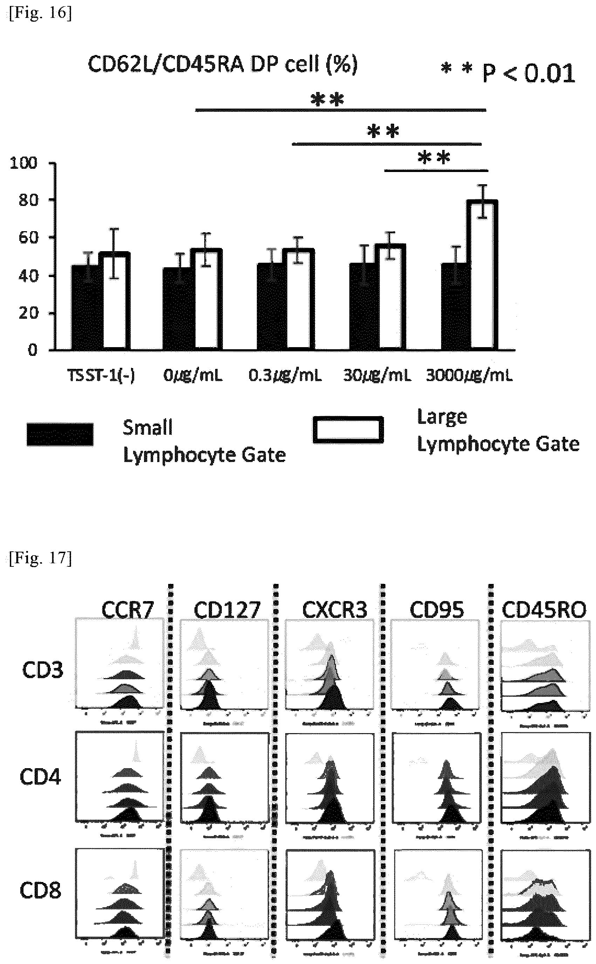

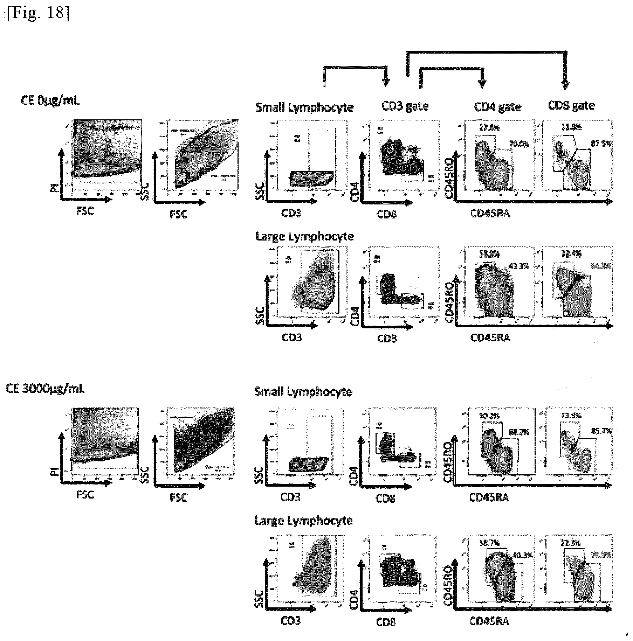

As mentioned above, it was demonstrated that the T cells stimulated by TSST-1 in the presence of CE comprised both non-proliferating cells and rapidly dividing cells (activated T cells). Thus, the phenotypes of these T cells were analyzed. As a result, it was found that the percentage of the T cell fraction expressing both CD45RA and CD62L was increased in the large lymphocyte gate in a CE concentration-dependent manner (Fig. 15 and Fig. 16). CD45RA and CD62L double positive cells indicate naive T cells. Hence, the expression levels (MFI) of markers of naive T cells or memory T cells, such as CCR7, CD127, CD95 and CXCR3, were further analyzed. As a result, the expression of all of these markers, and in particular, the expression of CD95, was increased or maintained in the double positive cells in the 3000 µg/ml CE culture solution (Fig. 17). This suggests that stem cell-like memory T cells (T_{SCM}) are comprised in the T cells stimulated by TSST-1 in the presence of CE. In addition, regarding CD4, most of the CD4 T cells also expressed CD45RO (Fig. 18), suggesting that these cells are not typical memory T cells (T_{SCM}), but that most of the CD8T cells were negative for CD45RO and contained typical Tscm.

### 5. Consideration

The aforementioned results showed that CE plays a role for regulating differentiation of the activated T cells stimulated with TSST-1. Specifically, it was suggested that CE activates effector T cells and also promotes their differentiation into early memory T cells having the same phenotype as that of T_{SCM} cells. This suggests that CE maintains memory cells having a cellular proliferative ability, and simultaneously can enhance the function of effector T cells.

The T cells stimulated with TSST-1 proliferated more quickly in the presence of a high concentration of CE. The percentage of effector T cells as Th1, Th2 and Th17 cells tended to increase in PBMCs. In addition, the expression of CD25 was increased in these T cells, suggesting that some T cells are extensively activated. Furthermore, in these T cells, a significant increase in IL-1β was observed, while the levels of TNF-α and IL-10 were significantly decreased. It was demonstrated that CE can regulate the inflammatory cytokine profiles of lymphocytes and/or innate immune cells.

Moreover, activated T cells did not increase the expression of PD-1. Herein, PD-1 is a late-activation marker, and PD-1-expressing cells are exhausted and apoptosis is induced by PD-L1/PD-1 signaling. Accordingly, the decrease of PD-1 suggests that exhaustion of T cells is suppressed in the presence of CE.

Meanwhile, in the large lymphocyte gate, the percentage of CD45RA and CD62L double positive T cells was increased (90% at maximum), and in these double positive T cells, the enhancement of other naive markers such as CCR7 was confirmed. Also, CD95, a memory marker, was enhanced in these double positive T cells, indicating that T_{SCM} cells may have been produced. Besides, CD8T cells did not express CD45RO, but most of the double positive CD4 T cells expressed CD45RO. CD45RO is not expressed in common T_{SCM} cells, but CD45RO is expressed in induced T_{SCM} cells, central memory T cells (T_{CM} cells), and effector memory T cells (T_{EM} cells). Therefore, it is likely that CE regulates differentiation of the T cells activated by stimulation with TSST-1 by inducing signals to allow the T cells to develop not only into central memory T cells (T_{CM} cells) and effector memory T cells (TE_{M} cells), but also into early memory T cells such as T_{SCM} cells.

### Industrial Applicability

The T cell differentiation-regulating agent of the present invention comprises a substance derived from microalgae as an active ingredient, and the present T cell differentiation-regulating agent is able to regulate differentiation of T cells activated by stimulation with TSST-1. Hence, preventive and therapeutic strategies for a decrease in the immune functions attended with overactivation of T cells can be provided. In addition, since the T cell differentiation-regulating agent of the present invention is able to suppress exhaustion and apoptosis of T cells, it provides novel therapeutic strategies for prevention of the occurrence of cytokine storms and suppression of a decrease in the immune functions. Moreover, since the active ingredient of the T cell differentiation-regulating agent of the present invention is an algal body of microalgae, high safety can also be expected.

## Claims

1. A T cell differentiation-regulating agent, comprising, as an active ingredient, an algal body of microalgae belonging to the genus Coccomyxa, a dry powder thereof, or an extract thereof.

2. The T cell differentiation-regulating agent according to claim 1, which has an action to promote proliferation of activated effector T cells, and an action to preserve memory T cells.

3. The T cell differentiation-regulating agent according to claim 1 or 2, which suppresses overactivation of T cells.

4. The T cell differentiation-regulating agent according to any one of claims 1 to 3, wherein the microalga is a Coccomyxa sp. KJ strain or a mutant strain thereof.

5. The T cell differentiation-regulating agent according to any one of claims 1 to 4, which is a medicament, a quasi-drug, a cosmetic product, or a miscellaneous product.

6. The T cell differentiation-regulating agent according to any one of claims 1 to 4, which is a food product, a food additive, or a feed.

7. A composition, comprising the T cell differentiation-regulating agent according to any one of claims 1 to 6.

8. The composition according to claim 7, which is a medicament, a quasi-drug, a cosmetic product, or a miscellaneous product.

9. The composition according to claim 7, which is a food product, a food additive, or a feed.
